# EUROPÄISCHE PATENTSCHRIFT

(11) **EP 4 022 186 B1**
(45) Veröffentlichungstag und Bekanntmachung des Hinweises auf die Patenterteilung: **23.08.2023**
(21) Anmeldenummer: 21710269.8
(22) Anmeldetag: 05.03.2021
(51) Int. Cl.: F04D 29/42, F04D 29/66, F04D 29/08, F04D 27/00, A61M 16/00, F04D 29/16

(54) **LÜFTER MIT DÄMPFENDEN UND DICHTENDEN ELEMENTEN ZWISCHEN VERSCHIEDENEN GEHÄUSETEILEN**
FAN WITH DAMPING AND SEALING ELEMENTS BETWEEN DIFFERENT CASING PARTS
VENTILATEUR AVEC ÉLÉMENTS AMORTISSANTS ET ÉTANCHÉIFIANTS ENTRE DIFFÉRENTES PARTIES DE CARTER

(30) Priorität: 05.05.2020 DE 102020002671
(43) Veröffentlichungstag der Anmeldung: 06.07.2022
(73) Patentinhaber: Drägerwerk AG & Co. KGaA, 23558 Lübeck (DE)
(72) Erfinder: CASPARY, René-Christian, 23558 Lübeck (DE)
(86) Internationale Anmeldenummer: PCT/EP2021/055567
(87) Internationale Veröffentlichungsnummer: WO 2021/223923

(56) Entgegenhaltungen:
- CN-U- 205 383 109
- DE-C- 197 769
- DE-C1- 19 715 581
- US-A1- 2014 325 789
- US-A1- 2018 064 894
- US-A1- 2019 038 866
- US-B1- 6 474 960

## Beschreibung

Die Erfindung betrifft einen Verdichter zur Förderung eines Luft- und/oder Gasstroms mit einem Gehäuse, in dessen Gehäuseinnenraum ein drehbar gelagertes und über eine Antriebswelle mit einem Elektromotor verbundenes Verdichterrad angeordnet ist. Bei einer Drehung des Verdichterrades wird der Luft- und/oder Gasstrom von einem stromaufwärts auf einer Saugseite des Verdichterrades angeordneten Einlass durch einen Strömungskanal zu einem stromabwärts auf einer Druckseite des Verdichterrades angeordneten Auslass gefördert. Derart gattungsgemäße Verdichter werden etwa in Beatmungs- oder Anästhesiegeräten eingesetzt, um einen Patienten mit dem jeweils benötigten Atemgastrom zu versorgen.

Häufig erfolgt die Versorgung von Beatmungs- oder Anästhesiegeräten mit dem jeweils benötigten Gasgemisch über die zentrale Gasversorgung eines Krankenhauses, an die die Geräte angeschlossen sind. Allerdings werden in zunehmendem Maße Gebläse mit geeigneten Verdichtern in Beatmungs- und Anästhesiegeräten verbaut, durch die ein Atemgasstrom, der Luft oder ein andersartig zusammengesetztes Gasgemisch aufweisen kann, mit den erforderlichen Zustandsparametern zum Patienten gefördert wird. In diesem Zusammenhang ist es sowohl denkbar, den Atemgastrom auf einem kontinuierlichen Druckniveau zu fördern oder das Druckniveau dynamisch zu variieren, um so etwa gezielte Beatmungsmodi oder Beatmungsmanöver zu realisieren.

Wesentlich für die in Beatmungs- oder Anästhesiegeräten eingesetzten Gebläse ist, dass diese über eine kompakte Bauform verfügen, einen Atemgasstrom erzeugen können, der die für die Beatmung des jeweiligen Patienten erforderlichen Zustandsparameter aufweist, und nur möglichst wenig Schall, der oftmals in Form von Körperschall durch Schwingungen von Motor- oder Gehäuseteilen hervorgerufen wird, emittieren. Da es regelmäßig nicht möglich ist, alle drei vorgenannten Auslegungskriterien in gleichem Maß zu erfüllen, beruht die Entwicklung und Auslegung der gattungsgemäßen Verdichter vielfach auf einer Optimierung, sodass jedes der drei Auslegungskriterien zumindest hinreichend erfüllt wird.

In diesem Zusammenhang ist ein gattungsgemäßes Gebläse mit einem Rotationsverdichter für ein Beatmungssystem aus der US 5,875,783 bekannt. Aufgrund der spezifischen Dimensionierung und Ausformung sowie der hierdurch realisierten Eigenschaften ist der beschriebene Verdichter besonders geeignet, um auf Druckschwankungen, die vor allem bei der Unterstützung eines zumindest teilweise spontan atmenden Patienten auftreten, zu folgen.

Ein weiteres Gebläse für Beatmungs- und Anästhesiegeräte ist aus der DE 197 14 644 C2 bekannt. Das Gebläse verfügt über einen Radialverdichter mit rückwärts gekrümmten Schaufeln. Aufgrund der speziellen Formung des Verdichterrades ist es möglich, bei entsprechender Ansteuerung des Elektromotors innerhalb einer Zeitspanne von nur wenigen Millisekunden einen großen Drehzahl- und Druckbereich abzudecken.

Weiterhin beschreibt die WO 2007/134405 A1 ein Gebläse, das sich durch geringe Schallemissionen auszeichnen soll. Wesentlich für das beschriebene Gebläse ist, dass eine Mehrzahl von Verdichterstufen strömungstechnisch in Reihen angeordnet sind und sämtliche strömungsführenden Bauteile von einem zusätzlichen Gehäuse umgeben sind.

Darüber hinaus sind aus der US 6,474,960 B1 und der US 2014/325789 A1 gattungsgemäße Verdichter bekannt, die jeweils über ein Gehäuse mit einem im Gehäuseinnenraum drehbar gelagertes Verdichterrad verfügen, das, angetrieben durch einen Elektromotor, einen Luft- oder Gasstrom fördert.

Ein weiterer Rotationsverdichter ist aus der DE 199 04 119 C2 bekannt. Der Antrieb des Verdichters erfolgt durch einen elektronisch kommutierten Gleichstrommotor, dessen Rotor durch einen mit dem Verdichterrad verbundenen Permanentmagneten gebildet ist, wobei der Rotor vom Stator des Gleichstrommotors durch einen Spalttopf hermetisch getrennt ist. Weiterhin ist das Verdichterrad mithilfe eines aerodynamischen Gas-Gleitlagers gelagert und von einem aus mehreren Komponenten zusammengesetzten Gehäuse umgeben. Mit Hilfe der beschriebenen technischen Lösung soll vor allem der direkte Kontakt zwischen drehenden Teilen des Elektromotors und dem Gehäuse verhindert werden.

Da die in der DE 199 04 119 C2 beschriebene Lösung über einen vergleichsweise aufwändigen Aufbau verfügt, sehen bekannte, hierzu alternative Lösungen ein zusätzliches Gehäuse, das sogenannte Sekundärgehäuse vor, das den Verdichter sowie das an diesen angrenzende, den Luft- oder Gasstrom leitende Spiralgehäuse, das auch als Primärgehäuse bezeichnet wird, umgibt.

Um bei Einsatz eines Sekundärgehäuses eine weitere Minimierung der Schallemission zu erreichen, werden vielfach Maßnahmen zur Verringerung von Schwingungsübertragungen getroffen. In diesem Zusammenhang ist es etwa bekannt, die aus Motor, Verdichterrad und Primärgehäuse gebildete Baueinheit mithilfe von flexiblen, weichen Aufnahmen oder Entkopplungselementen im Sekundärgehäuse zu haltern. Eine Schalldämmung wird in diesem Fall durch ein zusätzliches Gehäuse und weitere Dämmelemente realisiert, sodass letztendlich zwar die Lärmbelastung eines Patienten und des Pflegepersonals minimiert, dafür aber die Realisierung einer kompakten Verdichterbauform verhindert wird.

Problematisch an dem aus dem Stand der Technik bekannten, insbesondere in Beatmungs- und Anästhesiegeräten eingesetzten Verdichtern ist, dass deren Auslegung in Bezug auf die erforderlichen Leistungsparameter zwar möglich ist, dass allerdings zusätzlich auch eine kompakte Bauform und geringe Schallemissionen wesentliche Auslegungskriterien darstellen. Eine möglichst optimale Erfüllung aller dieser drei Auslegungskriterien stellt regelmäßig ein erhebliches Problem dar.

Ausgehend von den aus dem Stand der Technik bekannten Verdichtern und den zuvor geschilderten Problemen liegt der Erfindung die Aufgabe zu Grunde, eine technische Lösung anzugeben, die die Bereitstellung eines hinsichtlich der drei wesentlichen Auslegungskriterien, nämlich Leistungscharakteristik, Bauform und Schallemission, optimierten Verdichters ermöglicht. Es soll somit ein Verdichter geschaffen werden, der für den Einsatz in einem Beatmungs- und/oder Anästhesiegerät geeignet ist, und sich durch eine vergleichsweise kompakte Bauform, geeignete Leistungscharakteristik als auch durch geringe Schallemissionen auszeichnet. Hierbei ist es von großer Bedeutung, die Belastungen eines Patienten sowie des Pflegepersonals durch vom Verdichter ausgehende Schallemissionen, die etwa durch Schwingungen unterschiedlicher Komponenten versursacht werden, zumindest zu minimieren.

In Bezug auf die pneumatische Leistungscharakteristik ist für die anzugebende Lösung zu berücksichtigen, dass der Verdichter für den Einsatz in der Beatmung von Patienten einsetzbar sein soll, wobei in Abhängigkeit der jeweiligen Erkrankung eines Patienten spezifische Beatmungsparameter realisierbar sein sollen. Darüber hinaus sollte sich ein derartiger Verdichter durch einen vergleichsweise einfachen konstruktiven Aufbau, bei dessen Realisierung bekannte Konstruktionsprinzipien berücksichtigt werden, sowie möglichst geringe Herstellkosten auszeichnen. Im Weiteren ist es wünschenswert, wenn ein auf der Erfindung beruhender Verdichter möglichst geringe Strömungsverluste bei der Luft- oder Gasführung, zumindest in häufig zum Einsatz kommenden Betriebsbereichen, verursacht und wartungsfreundlich ausgeführt ist.

Die zuvor beschriebene Aufgabe wird mit einem Gebläse gemäß Anspruch 1 sowie einem Beatmungs- oder Anästhesiegerät nach Anspruch 13 gelöst. Vorteilhafte Ausführungsformen der Erfindung sind Gegenstand der abhängigen Ansprüche und werden in der folgenden Beschreibung unter teilweiser Bezugnahme auf die Figuren näher erläutert.

Die Erfindung betrifft einen Verdichter zur Förderung eines Luft- und/oder Gasstroms mit einem Gehäuse, in dessen Gehäuseinnenraum ein drehbar gelagertes, über eine Antriebswelle mit einem Elektromotor verbundenes Verdichterrad angeordnet ist, bei dessen Drehung der Luft- und/oder Gasstrom von einem stromaufwärts auf einer Saugseite des Verdichterrades angeordneten Einlass durch einen Strömungskanal zu einem stromabwärts auf einer Druckseite des Verdichterrades angeordneten Auslass gefördert wird. Ein erfindungsgemäß ausgezeichneter Verdichter zeichnet sich dadurch aus, dass das im Gehäuseinnenraum angeordnete Verdichterrad einerseits zumindest bereichsweise von dem für den radial nach außen verdrängten Luft- und/oder Gasstrom ein Sammelgehäuse darstellendes Gehäuse, das spiralförmig ausgebildet und zumindest teilweise auf der Druckseite des Verdichterrades angeordnet ist, und andererseits von einem Deckelelement, das zumindest teilweise auf der Saugseite des Verdichterrades angeordnet und wenigstens abschnittsweise durch einen Spalt vom Verdichterrad getrennt ist, umgeben ist. Hierbei ist zum einen zwischen dem Deckelelement und dem Sammelgehäuse und zum anderen zwischen einem mit dem Verdichterrad zumindest mittelbar verbundenen Funktionsbauteil und dem Sammelgehäuse jeweils wenigstens ein Entkopplungselement zur Schwingungsdämpfung und wenigstens teilweisen Abdichtung des Gehäuseinnenraums gegenüber einer Umgebung angeordnet.

Wesentlich für die erfindungsgemäße Lösung ist, dass der wenigstens zeitweise mit dem zu fördernden Luft- oder Gasstrom gefüllte Gehäuseinnenraum, in dem das drehbar gelagerte Verdichterrad angeordnet ist, wenigstens bereichsweise von einer Einhausung, die zumindest zwei Komponenten aufweist, nämlich ein spiralförmig ausgebildetes Sammelgehäuse, also ein sogenanntes Spiralgehäuse, sowie ein Deckelelement, umgeben ist, wobei sowohl zwischen dem Sammelgehäuse und dem Deckelelement als auch zwischen dem Sammelgehäuse und einem an dieses angrenzenden Funktionsbauteil, das zumindest mittelbar mit dem Elektromotor und/oder dem Verdichterrad verbunden ist, wenigstens ein Entkopplungselement zur Schwingungsdämpfung und wenigstens teilweisen Abdichtung des Gehäuseinnenraums gegenüber einer Umgebung vorgesehen ist. Das spiralförmig ausgebildete Sammelgehäuse ist somit sowohl gegenüber dem Funktionsbauteil als auch dem Deckelelement, das stromaufwärts auf der Saugseite des Verdichterrades angeordnet ist, schwingungsmäßig entkoppelt, sodass beispielsweise durch Einsatz eines elastischen Entkopplungselementes eine Dämpfung auftretender Schwingungen erreicht wird. So wird eine Übertragung von Schwingungen von dem Deckelelement auf das Spiralgehäuse aber auch von dem angrenzenden Funktionsbauteil auf das Spiralgehäuse zuverlässig verhindert oder zumindest minimiert. Das den Gehäuseinnenraum zumindest bereichsweise begrenzende Sammelgehäuse ist auf diese Weise, insbesondere gegenüber dem Elektromotor und dem mit diesem zumindest mittelbar verbundenen Verdichterrad in Bezug auf eine mögliche Schwingungsübertragung entkoppelt und gewährleistet damit eine Verminderung der abgestrahlten Schallemissionen, ohne dass hierfür ein zusätzliches Sekundärgehäuse, welches das den Gehäuseinnenraum zumindest bereichsweise begrenzende Sammelgehäuse, das auch als Primärgehäuse, bezeichnet werden kann, umgibt, vorgesehen werden muss. Durch die gewählte Entkopplung wird effektiv die Übertragung von Vibrationen, die vom Elektromotor und/oder dem Verdichterrad erzeugt und von einem Funktionsbauteil, insbesondere einem Gehäuseteil, abgestrahlt werden, deutlich reduziert. Vor allem wirkt das Sammelgehäuse selbst nicht als Abstrahlfläche, da es von der Vibrationsquelle entkoppelt ist und stellt gemäß der erfindungsgemäßen technischen Lösung sogar einen Teil einer Kapselung des Verdichters dar. Das Sammelgehäuse übernimmt hierbei als Primärgehäuse die gleiche Funktion, wie es bei den aus dem Stand der Technik bekannten Lösungen die sogenannten Sekundärgehäuse tun. Wesentlich für die Erfindung ist somit, dass das Sammelgehäuse mittelbar über geeignete Entkopplungselemente, die die Übertragung von Schwingungen verhindern oder zumindest minimieren, an angrenzenden Bauteilen, die während des Betriebs des Verdichters zu Schwingungen angeregt werden können, befestigt ist. Durch das Vorsehen einer geteilten Einhausung für das Verdichterrad in Kombination mit flexiblen und gleichzeitig den Gehäuseinnenraum gegenüber der Umgebung abdichtenden Elementen zwischen den Einhausungsteilen, also zwischen dem Deckelelement und dem Sammelgehäuse und/oder zwischen dem Sammelgehäuse und einem angrenzenden Funktionsbauteil, ist es einerseits möglich, die Übertragung von Schwingungen, die vom Verdichterrad und/oder vom Elektromotor ausgehen, auf das Sammelgehäuse zu verhindern und andererseits dennoch Spaltverluste im Gehäuseinnenraum zwischen der Saug- und der Druckseite zu minimieren. Dies wird erfindungsgemäß dadurch erreicht, dass auf der Saugseite des Verdichterrades ein vom Sammelgehäuse durch ein Entkopplungselement separiertes Deckelelement angeordnet ist, das nur durch einen vergleichsweise kleinen Spalt vom drehbar gelagerten Verdichterrad getrennt ist. Da das erfindungsgemäß vorgesehene Deckelelement nicht, wie es aus dem Stand der Technik bekannt ist, über eine flexible Aufhängung mit dem Elektromotor verbunden ist, der seinerseits eine starre Verbindung über die Antriebswelle zum Verdichterrad aufweist, können bei Realisierung der Erfindung vergleichsweise kleine Toleranzen sichergestellt werden.

Die Einhausung des Gehäuseinnenraums mit dem darin drehbar gelagerten Verdichterrad wird erfindungsgemäß von einer Einhausung umgeben, die als Bauteile ein Deckelelement sowie ein Sammelgehäuse, das als Spiralgehäuse ausgebildet ist, aufweist. Bei dem an das Sammelgehäuse angrenzenden und über ein Kopplungselement verbundenen Funktionsbauteil handelt sich bevorzugt um ein Teil eines Sammelgehäuses und/oder einer Kapselung des Elektromotors, der das Verdichterrad antreibt. Ebenso ist es denkbar, dass das Funktionsbauteil zumindest mittelbar oder unmittelbar mit dem Elektromotor verbunden ist. Durch das elastische Entkopplungselement zwischen dem spiralförmig ausgebildeten Sammelgehäuse und dem Funktionsbauteil wird eine Übertragung von Schwingungen, die vom Elektromotor mit seinen bewegten Bauelementen, insbesondere der Antriebswelle mit dem daran befestigten Verdichterrad, während des Betriebs wenigstens zeitweise erzeugt werden, zuverlässig verhindert oder zumindest verringert. Vorzugsweise verfügt das Funktionsbauteil über eine kreis- oder ovalförmig ausgebildet Dichtfläche, an der das Entkopplungselement, das eine elastische, wenigstens teilweise flexible Verbindung zwischen dem Funktionsbauteil und dem Sammelgehäuse herstellt, befestigt ist. Das Entkopplungselement dient hierbei sowohl der Verhinderung oder zumindest Minimierung einer Schwingungsübertragung als auch einer Abdichtung des Gehäuseinnenraums, in dem sich der vom Verdichterrad geförderte, wenigstens teilweise verdichtete Luft- oder Gasstrom befindet, gegenüber der Umgebung.

In einer speziellen Ausführungsform der Erfindung ist das Deckelelement, das saugseitig des Verdichterrades angeordnet ist, in Form einer Scheibe ausgeführt. Hierbei verfügt die Scheibe im Scheibeninneren über einen Durchlass, der vorzugsweise einen kreisscheibenförmigen Querschnitt aufweist und durch den der aufgrund der Drehung des Verdichterrades angesaugte Luft- und/oder Gasstrom in den Einlass des Verdichters eintritt.

Gemäß einer besonderen Weiterbildung der Erfindung ist das Deckelelement wenigstens bereichsweise mittelbar oder unmittelbar mit einer Strömungsführungseinheit verbunden, die über wenigstens ein Leitelement, etwa in Form einer Leitschaufel, zur Führung des auf der Druckseite des Verdichterrades austretenden Luft und/oder Gasstroms verfügt. Von Vorteil ist es, wenn sich das Deckelelement über die Strömungsführungseinheit abstützt, wobei es in diesem Zusammenhang denkbar ist, dass das Deckelelement auf einer dem Spalt zwischen Deckelelement und Verdichterrad gegenüberliegenden Seite der Strömungsführungseinheit mit dem zwischen Spiralgehäuse und Funktionsbauteil angeordneten Entkopplungselement verbunden ist. In diesem Fall wird durch das Entkopplungselement sichergestellt, dass Vibrationen, die bei der Durchströmung des Stromführungselementes entstehen können, nicht auf das Sammelgehäuse übertragen werden, wodurch wiederum Schallemissionen, die durch die Abstrahlung von Körperschall vom Sammelgehäuse erzeugt werden, zuverlässig verhindert oder zumindest minimiert wird.

Gemäß einer speziellen Ausführungsform ist die Strömungsführungseinheit ringförmig ausgebildet und verfügt über eine Mehrzahl von entlang einer ringförmigen Umfangsfläche angeordneten Leitschaufeln. Hierbei ist das Verdichterrad derart im Inneren der Strömungsführungseinheit angeordnet und drehbar gelagert, dass der während des Betriebs wenigstens zeitweise aus dem Verdichterrad auf der Druckseite austretende Luft- und/oder Gasstrom wenigstens teilweise auf die Mehrzahl von Leitschaufeln auftrifft. Bevorzugt grenzen die feststehenden Leitschaufeln nicht direkt an das sich im Betrieb drehende Verdichterrad an, wobei es sich vorteilhaft anbietet, den Bereich zwischen dem Verdichterrad und den Leitschaufeln in Form eines Diffusors auszubilden. Vorzugsweise wird der Diffusor in Abhängigkeit der Strömungsparameter, die in wenigstens einem vergleichsweise häufig während des Verdichterbetriebs auftretenden Arbeitspunktes oder Arbeitsbereiches auftreten, ausgeführt.

Im Weiteren ist es von Vorteil, wenn das Verdichterrad über sich in radialer Richtung vom Außenumfang des Verdichterrades zumindest nahezu quer über zumindest weitgehend einen gesamten Querschnitt des Strömungskanals erstreckende Hauptschaufeln sowie jeweils wenigstens zwei zwischen den Hauptschaufeln angeordnete und kürzer als die Hauptschaufeln ausgeführte Zwischenschaufeln verfügt. Sowohl die Hauptschaufeln als auch die Zwischenschaufeln sind derart, beispielsweise gebogen und/oder gekrümmt ausgeführt, dass der zu fördernde Luft- und/oder Gasstrom möglichst effektiv verdichtet wird, vor allem Strömungsverluste minimiert werden.

Ein wie zuvor beschrieben ausgeführtes Verdichterrad zeichnet sich neben einer besonderen Effektivität bei der Verdichtung von Luft und/oder Gas vor allem durch vergleichsweise geringe Schallemissionen aus, da aufgrund der speziell ausgeführten Haupt- und Zwischenschaufeln eine Wirbelbildung im Inneren des Verdichterrades zuverlässig vermieden wird. Im Übrigen wird mit einem derart ausgebildeten Verdichterrad, bestehend aus Hauptschaufel und Zwischenschaufeln sichergestellt, dass trotz eines vergleichsweise geringen Bauraums eine hohe pneumatische Leistung erzielbar ist.

Gemäß einer besonders vorteilhaften Weiterbildung der Erfindung wird ein Verdichterrad verwendet, das einen Außendurchmesser von 29 bis 34 mm, bevorzugt von 30 bis 32 mm aufweist. Vorzugsweise sind zwischen acht und zehn, insbesondere zwischen neun und dreizehn Hauptschaufeln, zwischen denen jeweils wenigstens zwei Zwischenschaufeln angeordnet sind, an einem Verdichterrad vorgesehen.

Die Zwischenschaufeln erstrecken sich jeweils vom Außenumfang des Verdichterrades in Richtung der Mitte, sind allerdings kürzer als die Hauptschaufeln und bevorzugt ebenfalls gekrümmt und/oder gebogen. In einer ganz besonderen Ausführungsform sind die zwischen den Hauptschaufeln angeordneten Zwischenschaufeln in radialer Richtung unterschiedlich lang, verfügen über ein ungleiches Profil und/oder weisen verschieden große Schaufelflächen auf. Zwischen zwei bevorzugt gleichförmig ausgebildeten Hauptschaufeln sind gemäß dieser speziellen Ausführungsform somit wenigstens zwei Zwischenschaufeln, die entsprechend unterschiedlich ausgebildet sind, angeordnet.

In einer weiteren Ausführungsform der Erfindung ist stromaufwärts des Verdichterrades wenigstens ein Einlassschalldämpfer angeordnet. Bevorzugt weist der Einlassschalldämpfer wenigstens einen Spiralschalldämpfer auf. Auf vorteilhafte Weise sollte der Strömungsquerschnitt, der sich aus dem Abstand zwischen den Windungen der Spirale und der Spiralhöhe ergibt, bevorzugt etwa 25-35 %, bevorzugt etwa 30 % größer als der Einlassquerschnitt des Verdichterrades. Im Allgemeinen ist es von Vorteil, wenn der Abstand zwischen den einzelnen Windungen der Spirale geringgehalten wird, um so Schallemissionen, die durch Strömungsgeräusche verursacht werden und über den Einlass austreten können, zu minimieren. Eine Verringerung des Abstandes der Spiralwände führt bei einem definierten Strömungsquerschnitt allerdings zu einer Vergrößerung der resultierenden Bauhöhe der Spirale eines Spiralschalldämpfers. Um dennoch eine kompakte und möglichst flache Bauform realisieren zu können, verfügt gemäß einer besonderen Weiterbildung der Erfindung der Einlassschalldämpfer über einen Spiralschalldämpfer mit wenigstens zwei, zumindest teilweise ineinander angeordneten, separaten Spiralelementen. In diesem Zusammenhang ist es denkbar, dass die ineinander verschachtelten Spiralen den gleichen oder unterschiedliche Strömungsquerschnitte aufweisen. Bevorzugt wird der Abstand der Spiralwände bei Einsatz einer zweiten Spirale jedoch halbiert, was die Schalldämpfungscharakteristik eines Spiralschalldämpfers bei gleichen oder zumindest ähnlichen Außenabmessungen deutlich verbessert.

Ein Einlassschalldämpfer, in dem eine Dämpfung durch im Strömungskanal vorgesehene Querschnittsverengungen und Umlenkungen realisiert wird, beispielsweise ein Spiralschalldämpfer, kann allerdings nicht hinsichtlich einer maximalen Schalldämpfung optimiert werden, da sowohl Querschnittsverengungen als auch Umlenkungen zu einer Erhöhung des Strömungswiderstandes und damit zu einer ungewünschten Verringerung der pneumatischen Leistungsfähigkeit führen. Demgegenüber gewährleisten große Strömungsquerschnitte einen geringen strömungstechnischen Widerstand und damit eine hohe Leistungsfähigkeit. Die Auslegung eines Einlassschalldämpfers erfordert daher stets einen Kompromiss zwischen einer Minimierung der Schallemissionen und der pneumatischen Leistungsfähigkeit. Ein theoretisch optimaler Schalldämpfer würde sich dadurch auszeichnen, dass der Strömungsquerschnitt bei einem maximalen Volumenstrom des vom Verdichter geförderten Luft- oder Gasstroms ebenfalls maximal groß ist, mit einer steigenden Drosselung des Verdichters kleiner wird und bei vollständiger Drosselung, bei der der Volumenstrom somit einem Betrag von Null annimmt, den Einlass vollkommen verschließt.

Gemäß einer speziellen Weiterbildung der Erfindung wird die zuvor beschriebene Funktionalität zumindest näherungsweise dadurch erreicht, dass ein Einlassschalldämpfer wenigstens ein selbsttätig schließendes Ventilelement aufweist, das in Abhängigkeit der am Ventil anliegenden Druckdifferenzen öffnet und schließt. Die Betätigung des Ventils erfolgt hierbei bevorzugt aufgrund der am Ventilteller anliegenden Druckdifferenz. Das Ventilelement ist daher auf vorteilhafte Weise in Form eines Rückschlagventils ausgeführt, das ein Rückströmen von Luft- und/oder Gas aus dem Strömungskanal des Verdichterrades zuverlässig verhindert. Vorzugweise verfügt das Ventilelement mit einem Ventilteller, der in Form einer Ventilmembran ausgeführt ist.

In einer weiteren besonderen Ausführungsform wird eine gewünschte Öffnungscharakteristik dadurch eingestellt, dass eine Steifigkeit einer als Ventilteller verwendeten Ventilmembran, eine Anordnung einer Ventilmembran und/oder eine Befestigung einer Ventilmembran bedarfsgerecht gewählt wird.

Bevorzugt ist das ein Ventilelement eines Einlassschalldämpfers derart ausgeführt, dass es einen maximalen Strömungsquerschnitt zwischen einem Ventilteller und einem Ventilsitz bei maximaler Volumenstrom, also bei maximaler pneumatischer Leistung des Verdichters, und einen minimalen Strömungsquerschnitt bei maximal gedrosseltem Betrieb des Verdichters freigibt. Hierdurch wird während des Betriebs eine maximal mögliche Minimierung des durch den Einlass emittierten Schalls und damit eine maximale Schalldämmung erreicht.

Gemäß einer ganz besonderen Weiterbildung ist in diesem Zusammenhang vorgesehen, dass eine Mehrzahl derartiger Ventilelemente mit unterschiedlichen Öffnungscharakteristika in Reihe geschaltet werden, sodass die Kennlinie des Verdichters bedarfsgerecht optimiert werden kann, beispielsweise eine konstante Verdichtung über einen vorgegebenen Volumenstrombereich realisiert wird.

Wenigstens eines der zuvor beschriebenen Ventilelemente kann auf vorteilhafte Weise allein oder gemeinsam mit einem Spiralschalldämpfer, der gemäß einer der zuvor beschriebenen Ausführungsformen gestaltet ist, zur Minimierung des über den Einlass austretenden Schalls eingesetzt werden, insbesondere um Schallemissionen, die vom Verdichterrad erzeugt und entgegen der Strömungsrichtung des geförderten Luft- und/oder Gasstroms über den Einlass austreten würden, zu verhindern oder zumindest zu verringern. Diese Schallemissionen über den Einlass werden üblicherweise stärker, sofern das Verdichterrad auf einem gleichbleibenden Drehzahlniveau gedrosselt wird. Hieraus folgt, dass in einem Betriebspunkt, in dem der Verdichter nicht durchströmt wird, die Schallemission einen maximalen Wert annimmt.

Im Folgenden wird die Erfindung ohne Beschränkung des allgemeinen Erfindungsgedankens anhand spezieller Ausführungsbeispiele unter Bezugnahme auf die Figuren näher erläutert. Dabei zeigen:
- Fig. 1:: Schematische Schnittansicht eines aus dem Stand der Technik bekannten Verdichters mit Primär und Sekundärgehäuse;
- Fig. 2:: Schematische Schnittansicht eines erfindungsgemäß ausgeführten Verdichters;
- Fig. 3:: Schnittansicht eines erfindungsgemäß ausgeführten Verdichters;
- Fig. 4:: Schnittansicht einer Strömungsführungseinheit eines erfindungsgemäß ausgeführten Verdichters;
- Fig. 5:: Perspektivenansicht sowie Draufsicht auf ein Verdichterrad eines erfindungsgemäß ausgeführten Verdichters;
- Fig. 6:: Perspektivische Darstellungen eines als Spiralschalldämpfer ausgeführten Einlassschalldämpfers mit einer Teilspirale;
- Fig. 7: Perspektivische Darstellungen eines als Spiralschalldämpfer ausgeführten Einlassschalldämpfers mit zwei ineinander verschachtelten Teilspiralen sowie
- Fig. 8:: Schnittdarstellungen eines Einlassschalldämpfers mit einem Rückschlagventil in geöffneter und geschlossener Betriebsstellung.

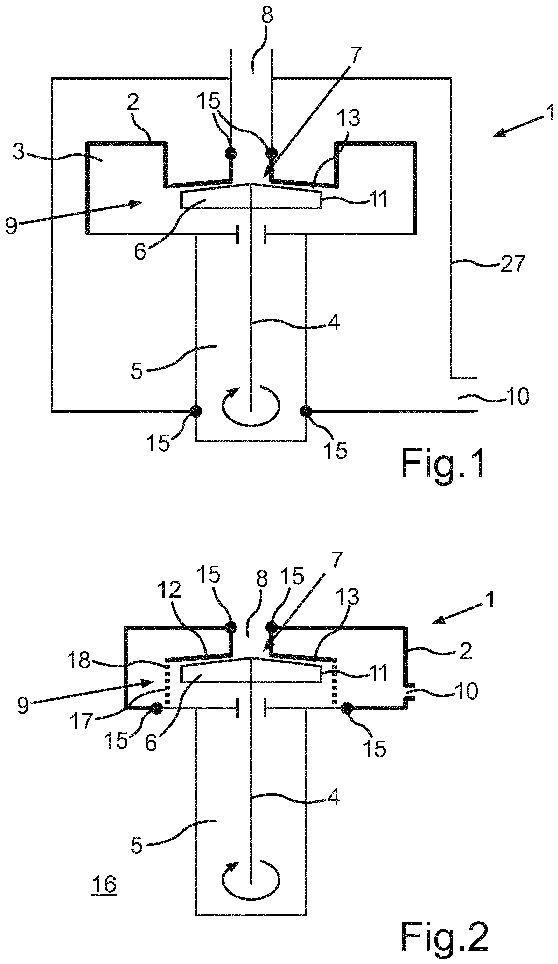

Fig. 1 zeigt zunächst einen Verdichter 1 für ein Beatmungs- oder Anästhesiegerät, wie er aus dem Stand der Technik bekannt ist. Der Verdichter 1 verfügt über einen Elektromotor 5 der ein Verdichterrad 6 über eine Antriebswelle 4 in Abhängigkeit der für die Beatmung eines Patienten erforderlichen Leistungsparameter antreibt. Das Verdichterrad ist in einem als Spiralgehäuse ausgeführten inneren Sammelgehäuse 2, das auch als Primärgehäuse bezeichnet werden kann, drehbar gelagert, in das während des Betriebs durch einen zentralen Einlass 8 Luft eintritt. Das Verdichterrad 6 ist als Radialverdichterrad ausgeführt, das in Abhängigkeit der benötigten pneumatischen Leistung direkt von dem Elektromotor 5 angetrieben wird.

Während des Betriebs wird Luft über einen zentralen Einlass 8 angesaugt, radial durch die Rotation des Verdichterrades 6 nach außen verteilt, in dem spiralförmigen Sammelgehäuse 2 gesammelt und zum Auslass 10 gefördert. Um während des Betriebs Schallemissionen möglichst gering zu halten, ist der Verdichter von einem zweiten Gehäuse 27, dem sogenannten Sekundärgehäuse, umgeben, das ebenfalls über einen Einlass sowie einen Auslass für den zu fördernden Luft- und/oder Gasstrom verfügt. Um die Übertragung von Vibrationen zu verhindern, ist die Einheit aus Elektromotor 5, Verdichterrad 6 und Primärgehäuse 2 mit flexiblen, weichen Entkopplungselementen 15 im Sekundärgehäuse 27 gehaltert. Das zusätzliche Gehäuse in Form des Sekundärgehäuses 27 ist vor allem daher erforderlich, da die Verbindungen zwischen Elektromotor 5 und Verdichterrad 6 sowie zwischen Elektromotor 5 und Primärgehäuse 2 starr ausgebildet sind, sodass sich Vibrationen, die betriebsbedingt vor allem bei hohen Motordrehzahlen erzeugt werden, je nach Ausführung der Verbindung nahezu ungestört auf alle damit verbundenen Gehäuseteile übertragen und so in die Umgebung 16 abgestrahlt werden. Eine derartige Konstruktion mit zwei separaten Gehäusen 2, 27 schließt eine kompakte Bauweise eines entsprechenden Verdienstes aus.

Im Übrigen ist Fig. 1 zu entnehmen, dass das Primärgehäuse 2 als einteiliges Sammelgehäuse den Gehäuseinnenraum 3 mit dem darin angeordneten Verdichterrad 6 umgibt. Hierbei befindet sich auf der Saugseite 7 des Verdichterrades 6 zwischen einem Deckelbereich des Primärgehäuses 2 und dem Verdichterrad 6 ein geringer Spalt 13. Dieser Spalt 13 muss möglichst geringgehalten werden, um eine Bypassströmung von der Druckseite 9 zur Saugseite 7 zu verhindern oder zumindest zu minimieren, da diese Bypassströmung die Leistungsfähigkeit des Verdichters reduziert. Andererseits muss der Spalt 13 derart dimensioniert werden, dass eine Berührung zwischen dem Verdichterrad 6 und dem Deckelbereich des Sammelgehäuses 2 in jedem Betriebszustand, insbesondere auch bei Erhöhung der Drehzahl und/oder einer Erwärmung des Verdichterrades 6, zuverlässig verhindert wird. Hieraus ergibt sich unmittelbar, dass der Widerspruch zwischen den Anforderungen für eine minimale Höhe des Spalts 13 zwischen Deckelbereich des Sammelgehäuses 2 und Verdichterrad 6 und der hierfür erforderlichen fixen Position des Deckelbereichs des Sammelgehäuses einerseits sowie der flexiblen Aufhängung eines Sammelgehäuses zur Gewährleistung einer möglichst effektiven Schalldämmung andererseits ein erhebliches konstruktives Problem darstellt.

In Fig. 2 ist in einer schematischen Schnittansicht ein erfindungsgemäß ausgeführter Verdichter dargestellt. Der Verdichter verfügt über ein in einem Gehäuseinnenraum 3 drehbar gelagertes Verdichterrad 6, das von einem Elektromotor 5 über eine Antriebswelle 4 angetrieben wird. Das Verdichterrad 6 wird in Abhängigkeit des benötigten Luft- und/oder Gasstroms vom Elektromotor 5 gedreht, wodurch der Luft- und/oder Gasstrom von dem stromaufwärts auf einer Saugseite 7 des Verdichterrades 6 angeordneten Einlass 8 durch einen Strömungskanal 11 zu einem stromabwärts auf der Druckseite 9 des Verdichterrades 6 angeordneten Auslass 10 gefördert wird.

Der Gehäuseinnenraum 3 sowie das darin angeordnete Verdichterrad 6 ist von einem spiralförmig ausgebildeten Sammelgehäuse 2, das daher auch als Spiralgehäuse bezeichnet werden kann, sowie einem scheibenförmigen Deckelelement 12 umgeben, wobei das Deckelelement 12 den Gehäuseinnenraum 3 insbesondere auf der Saugseite 7 und das Sammelgehäuse 2 den Gehäuseinnenraum 3 vor allem auf der Druckseite 9 begrenzt.

Erfindungsgemäß wird somit die den Gehäuseinnenraum 3 umgebenden Einhausung zweiteilig, nämlich aus einem spiralförmigen Sammelgehäuse 2 und einem scheibenförmigen Deckelelement 12 gebildet. Um die Übertragung von Schwingungen ausgehend vom Verdichterrad 6 und/oder vom Elektromotor 5 auf das Sammelgehäuse 2 zu vermeiden, sind Entkopplungselemente 15 sowohl zwischen dem Deckelelement 12 und dem Sammelgehäuse 2 als auch zwischen dem Sammelgehäuse 2 und einem Funktionsbauteil 14, bei dem es sich in diesem Fall um ein Teil des Motorgehäuses handelt, angeordnet. Die den Gehäuseinnenraum 3 mit dem darin angeordneten Verdichterrad 6 umgebende Einhausung ist somit in zwei Teile aufgeteilt worden, nämlich ein Deckelement 12 oberhalb des Verdichterrades 6 sowie das spiralförmige Sammelgehäuse 2, das auch als Spiralgehäuse oder Volute bezeichnet werden kann. Gemäß der in Fig. 2 gezeigten Ausführungsform ist das Deckelelement 12 scheibenförmig ausgeführt, wobei sich der Einlass 8 für den Luft und/oder Gasstrom mittig innerhalb der Scheibe befindet. Auf der Einlassseite ist das Deckelement 12 über ein flexibles Entkopplungselement mit dem Sammelgehäuse 2 verbunden, sodass eine Übertragung von Schwingungen von dem Deckelelement 12 zuverlässig verhindert oder zumindest minimiert wird. Des Weiteren wird mit Hilfe des in diesem Bereich vorgesehenen Entkopplungselements 15 eine Abdichtung des Gehäuseinnenraums 3 gegenüber der Umgebung sichergestellt.

Auf der dem Einlass 8 abgewandten Seite des Deckelelements 12 stützt sich dieses auf einer Strömungsführungseinheit 17 ab. Die Strömungsführungseinheit 17 stützt sich auf einer dem Deckelelement 12 abgewandten Seite wiederum mittelbar oder unmittelbar auf einem Funktionsbauteil 14, beispielsweise einem Gehäusebauteil des Elektromotors 5, ab. Auf diese Weise ist sichergestellt, dass das Deckelelement 12 einerseits mit Hilfe des Entkopplungselements 15 gegenüber dem Sammelgehäuse 2 schwingungsentkoppelt ist und andererseits eine starre Verbindung mit dem Funktionsbauteil 14 vorhanden ist, sodass sich ein vergleichsweise kleiner Spalt 13 zwischen dem Deckelelement 12 und dem Verdichterrad 6 realisieren lässt, ohne dass die Gefahr von Berührungen während des Betriebs besteht.

Die Strömungsführungseinheit 17, auf der sich das Deckelelement 12 abstützt ist gemäß der in Fig. 2 gezeigten Ausführungsforum auf der Druckseite des Verdichterrades 6 angeordnet, sodass der aus dem Verdichterrad 6 austretende Luft- und/oder Gasstrom auf die Strömungsführungseinheit 17 auftrifft und zumindest teilweise von dieser umgelenkt wird. Die gezeigte Strömungsführungseinheit 17 verfügt über feststehende Leitschaufeln 18, die nicht direkt an das sich im Betrieb drehende Verdichterrad 6 angrenzen. Der Bereich zwischen dem Verdichterrad 6 und den Leitschaufeln 18 ist für den am häufigsten genutzten Arbeitsbereich des Verdichters in Form eines Diffusors ausgebildet.

Wie der schematischen Darstellung in Fig. 2 deutlich zu entnehmen ist, sind Trennstellen zwischen dem Deckelelement 12 und dem Sammelgehäuse 2 sowie zwischen dem Sammelgehäuse 2 und dem Funktionsbauteil 14, das hier einen Teil des Motorgehäuses bildet, über Entkopplungselemente 15 verbunden, die einerseits eine schwingungstechnische Entkopplung der beidseitig angrenzenden Bauteile sicherstellen und andererseits den Druck beaufschlagten Gehäuseinnenraum 3 gegen die Umgebung 16 abdichten. Durch die vorgesehene Entkopplung werden Schwingungen, die insbesondere vom Elektromotor 5 erzeugt werden, nicht auf das den Gehäuseinnenraum 3 unmittelbar begrenzende Sammelgehäuse 2 übertragen oder die Schwingungsübertragung zumindest erheblich reduziert. Gemäß dem in Fig. 2 gezeigten Ausführungsbeispiel der Erfindung übernimmt das Sammelgehäuse 2 die Funktion, die bei den aus dem Stand der Technik bekannten technischen Lösungen das als zusätzliche Einhausung vorgesehene Sekundärgehäuse 27, wie es in Fig. 1 dargestellt ist, übernimmt. Aufgrund des Vorsehens der erfindungsgemäßen technischen Lösung kann ein Verdichter mit vergleichsweise geringen Abmessungen bereitgestellt werden, dessen pneumatische Leistung bei geeigneter Dimensionierung und Ausführung der Leitschaufeln 18 und des Diffusors für einen gewählten Arbeitsbereich gegenüber bekannten Lösungen sogar erhöht werden kann.

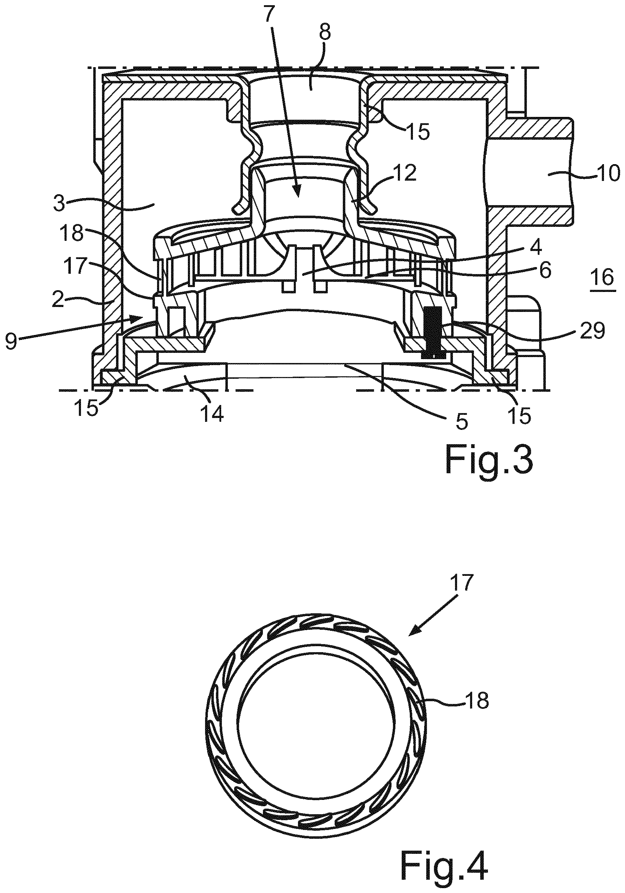

Fig. 3 zeigt eine Schnittansicht eines erfindungsgemäß ausgeführten Verdichters mit einem spiralförmigen Sammelgehäuse 2, einem Deckelelement 12 und Entkopplungselementen 15. Im Gehäuseinnenraum 3 ist das drehbar gelagerte Verdichterrad 6 angeordnet, das über eine Antriebswelle 4 von einem Elektromotor 5 angetrieben wird., Erfindungswesentlich ist wiederum, dass die Einhausung für den Gehäuseinnenraum 3 sowie das darin drehbar gelagerte Verdichterrad 6 aus dem Sammelgehäuse 2 sowie einem Deckelelement 12 gebildet wird. Sowohl zwischen dem Sammelgehäuse 2 und dem Deckelement 12 als auch zwischen dem Sammelgehäuse 2 und einem Funktionsbauteil 14 sind Entkopplungselemente 15 vorgesehen, die einerseits eine schwingungstechnische Entkopplung der jeweils an das Entkopplungselement 15 angrenzenden Bauteile und andererseits eine Abdichtung des Druck beaufschlagten Gehäuseinnenraums 3 gegenüber der Umgebung 16 gewährleisten. Das motorseitig angeordnete Entkopplungselement 15 ist derart ausgebildet, dass auch an einer Trennstelle zwischen dem Sammelgehäuse 2 und einem Funktionsbauteil 14, hier einem Teil des Gehäuses des Elektromotors 5, eine Schwingungsentkopplung realisiert wird. Durch dieses Entkopplungselement 15 wird somit zuverlässig sichergestellt, dass vom Verdichterrad 6 und insbesondere vom Elektromotor 5 erzeugte Vibrationen nicht oder nur geringfügig auf das den Gehäuseinnenraum 3 umgebende Sammelgehäuse 2 übertragen werden. Die Abstrahlung von störendem Schall wird somit zuverlässig vermieden oder zumindest erheblich verringert. Die Strömungsführungseinheit 17 ist motorseitig zumindest mittelbar über zumindest eine Schraube 29 mit dem Funktionsbauteil 14 verbunden. Auf vorteilhafte Weise übernimmt diese Schraube 29 die Funktion einer Verstellschraube, sodass durch geeignetes Anziehen und/oder Lösen der Schraube 29 die Breite des Spaltes 13 zwischen Deckelelement 12 und Strömungsführungseinheit 17 einstellbar ist.

Im Übrigen ist bei der in Fig. 3 gezeigten Baueinheit auf der Saugseite 7 ein Einlass 8 vorgesehen, durch den bei Drehung eines im Gehäuseinnenraum 3 angeordneten Verdichterrades 6 ein Luft- und/oder Gasstrom in Richtung des Verdichterrades 6 strömt. Bei Drehung des Verdichterrades 6 wird dieser angesaugte Luft- und/oder Gasstrom schließlich vom Verdichterrad 6 radial nach außen gedrückt und durch die Leitschaufeln 18 des Strömungsführungselementes 17, das mit dem Deckelement 12 verbunden oder alternativ einteilig mit diesem ausgeführt ist, radial nach außen in Richtung der Wand des Sammelgehäuses 2 geleitet. Durch einen Auslass 10 tritt der verdichtete Luft- und/oder Gasstrom anschließend aus dem Sammelgehäuse 2 des Verdichters aus und wird beispielsweise einem maschinell beatmeten Patienten zugeleitet.

Das auf der Saugseite vorgesehene Deckelelement ist scheibenförmig ausgeführt, wobei sich der Einlass 8 zentral in der Scheibenmitte befindet. Das Deckelement 12 stützt sich über die Strömungsführungseinheit 17 nach unten auf dem Funktionsbauteil 14, bei dem es sich in diesem Fall um ein Gehäuseteil des Elektromotors 5 handelt, ab. Im Bereich des Einlasses 8 ist ein Entkopplungselement 15 an der Trennstelle zwischen dem Deckelelement 12 und dem Sammelgehäuse 2 vorgesehen. Dieses Entkopplungselement 15 stellt einerseits sicher, dass Vibrationen nicht auf das Sammelgehäuse 2 übertragen werden und andererseits gewährleistet es eine Abdichtung des druckbehafteten Gehäuseinnenraums 3 gegenüber der Umgebung 16.

Die Strömungsführungseinheit 17 verfügt auf der Druckseite 9 über eine Mehrzahl von radial über den Umfang verteilten Leitschaufeln 18, die den vom Verdichterrad 6 radial nach außen gedrückten Luft- und/oder Gasstrom auf geeignete Weise umlenken. Der Bereich zwischen der Druckseite des Verdichterrades 6 und den Leitschaufeln 18 ist für einen Arbeitsbereich, der häufig auftritt, als Diffusor ausgebildet. Fig. 4 zeigt eine derartige spezielle Strömungsführungseinheit 17, die ringförmig ausgebildet ist und eine Mehrzahl von über den äußeren Umfang verteilten Leitschaufeln 18 aufweist, in einer perspektivischen Schnittdarstellung. Die Schnittebene verläuft hierbei horizontal durch die Strömungsführungseinheit 17. Die gezeigte Strömungsführungseinheit 17 ist derart ausgeführt, dass sich das erfindungsgemäß vorgesehene Deckelelement 12 darauf abstützen kann. Die Strömungsführungseinheit 17 verfügt hierfür über eine geeignete Anlagekontur oder kann alternativ einteilig mit dem Deckelelement 12 ausgeführt sein. In jedem Fall stellt das Strömungsführungselement 17 die mittelbare oder unmittelbare Verbindung zwischen dem Deckelelement 12 und dem motorseitig angeordneten Funktionsbauteil 14 her.

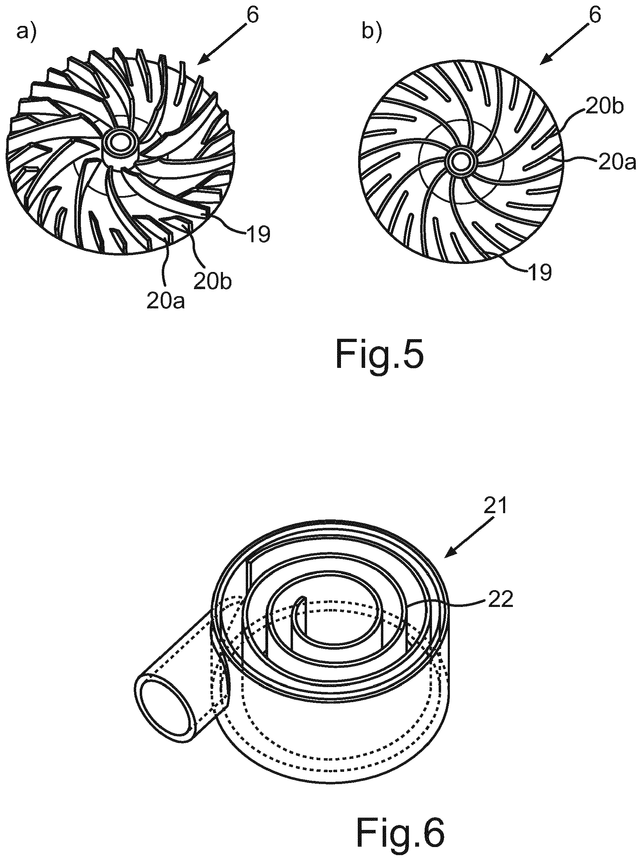

Fig. 5 zeigt in einer Perspektivansicht a) sowie einer Draufsicht b) ein speziell ausgebildetes Verdichterrad 6, wie es auf vorteilhafte Weise für einen erfindungsgemäß ausgebildeten Verdichter verwendet werden kann. Während der Drehung dieses Verdichterrades 6 wird ein Luft- und/oder Gasstrom mithilfe der Schaufeln 19, 20 radial nach au-ßen gefördert. Das gezeigte Verdichterrad 6 verfügt zunächst über Hauptschaufeln 19, die sich von einem äußeren Umfang des Verdichterrades zumindest nahezu vollständig bis in die Mitte des Verdichterrades, wo dieses an der Antriebswelle eines Elektromotors befestigt werden kann, erstrecken. Gemäß den in Fig. 5 gezeigten Ausführungsbeispiel verfügt das Verdichterrad 6 über 10 Hauptschaufeln 19. Zwischen den Hauptschaufeln 19 sind gemäß der in Fig. 5 gezeigten Ausführungsform jeweils zwei Zwischenschaufeln 20 angeordnet, die unterschiedlich lang ausgebildet sind, wobei in montiertem Zustand die längere Zwischenschaufeln 20a der Saugseite und die kürzeren Zwischenschaufel 20b der Druckseite des Verdichters zugewandt sind. In dem hier beschriebenen Ausführungsbeispiel haben die längeren Zwischenschaufeln 20a eine Länge, die etwa 40% der Länge der Hauptschaufeln 19 beträgt, während die Länge der kürzeren Zwischenschaufeln 20b nur etwa 30% der Länge der Hauptschaufeln 19 beträgt.

Aufgrund einer derartigen Ausführung der Zwischenschaufeln 20 wird auf vorteilhafte Weise eine Wirbelbildung zwischen den Hauptschaufeln 19 verhindert oder zumindest stark minimiert, sodass die ansonsten hierdurch verursachten Schallemissionen ebenfalls wenigstens verringert werden.

Das in Fig. 5 gezeigte Verdichterrad verfügt bevorzugt über einen Außendurchmesser von 30 bis 32 mm. Ferner ist es von Vorteil, wenn 9 bis 13 Hauptschaufeln 19 vorgesehen sind, zwischen denen jeweils, wie zuvor erläutert, zwei unterschiedlich dimensionierte Zwischenschaufeln 20 angeordnet werden.

Von Vorteil ist es, wenn die maximale Höhe der Hauptschaufeln 19 einen Wert zwischen 5 und 6 annimmt und die Oberkante der Hauptschaufeln 19 zumindest abschnittsweise in Richtung des Außenumfangs des Verdichterrades 6 geneigt ist. Ein Winkel zwischen 75 und 80° erscheint hier sinnvoll. Wesentlich für den effektiven betrieb eines Verdichterrades 6 ist vor allem die Ausführung des Schaufelkanals. Gemäß der gezeigten Ausführungsform sind die Abmessungen derart gewählt, dass der Austrittsquerschnitt etwa 30% größer als der Eintrittsquerschnitt ist.

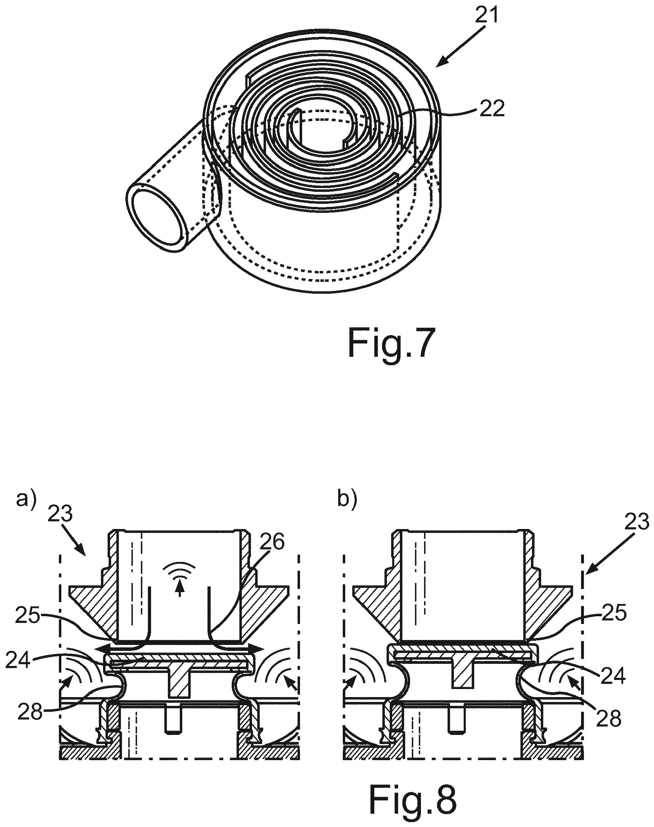

In Fig. 6 ist ein als Spiralschalldämpfer mit einem darin angeordneten Spiralelement 22 ausgeführter Einlassschalldämpfer 21 dargestellt. Durch die Verringerung des Strömungsquerschnittes im Inneren des Einlassschalldämpfers 21 sowie die Umlenkung der Strömung erfolgt eine Minimierung der in Richtung des Einlasses des Einlassschalldämpfers 21 austretenden Schallemissionen. Der Strömungsquerschnitt des Einlassschalldämpfers 21 wird derart dimensioniert, dass dieser etwa 30 % größer ist als der Einlassquerschnitt des Verdichters. Wird der Abstand zwischen den einzelnen Windungen des Spiralelements 22 geringgehalten, kann die Emission der über den Einlass eines Verdichters rückwärts austretenden Strömungsgeräusche minimiert werden. In Abhängigkeit der jeweils benötigten pneumatischen Leistung eines Verdichters stellt die zu starke Verkleinerung des Strömungsquerschnitts im Einlassschalldämpfer allerdings einen erheblichen Nachteil dar. Um trotz eines verringerten Wandabstands im Spiralelement 22 einen definierten Strömungsquerschnitt sicherzustellen müsste somit die resultierende Bauhöhe eines Einlassschalldämpfers 21 vergrößert werden, sodass dieser schnell vergleichsweise groß würde. Um eine kompakte und flache Bauform eines Einlassschalldämpfers zu erreichen, lässt sich daher auf bevorzugte Weise das im Inneren vorgesehene Spiralelement 22, wie in Fig. 7 dargestellt, in zwei Teilspiralen zerlegen, die ineinander verschachtelt sind. Das in Fig. 7 gezeigte Spiralelement, das aus zwei Teilspiralen besteht, hat denselben Strömungsquerschnitt wie das in Fig 6 dargestellte. Allerdings wurde der Wandabstand halbiert, sodass die Schalldämpfungscharakteristik für über den Einlass austretende Strömungsgeräusche bei ähnlichen Au-ßenabmessungen des Einlassschalldämpfers 21 deutlich verbessert wird.

Ein Einlassschalldämpfer 21, in dem eine Dämpfung durch im Strömungskanal vorgesehene Querschnittsverengungen und Umlenkungen realisiert wird, beispielsweise ein Spiralschalldämpfer wie er in den Figuren 6 und 7 gezeigt ist, kann allerdings nicht hinsichtlich einer maximalen Schalldämpfung optimiert werden, da sowohl Querschnittsverengungen, Vergrößerungen der Länge des Strömungskanals als auch Umlenkungen zu einer Erhöhung des Strömungswiderstandes und damit zu einer ungewünschten Verringerung der pneumatischen Leistungsfähigkeit führen. Demgegenüber gewährleisten große Strömungsquerschnitte und kurze Strömungskanäle einen geringen strömungstechnischen Widerstand und damit eine hohe Leistungsfähigkeit. Die Auslegung eines Einlassschalldämpfers erfordert daher stets einen Kompromiss zwischen einer Minimierung der Schallemissionen und der pneumatischen Leistungsfähigkeit. Ein theoretisch optimaler Schalldämpfer würde sich dadurch auszeichnen, dass der Strömungsquerschnitt bei einem maximalen Volumenstrom des vom Verdichter geförderten Luft- oder Gasstroms ebenfalls maximal groß ist, mit einer steigenden Drosselung des Verdichters kleiner wird und bei vollständiger Drosselung, bei der der Volumenstrom somit einem Betrag von Null annimmt, den Einlass vollkommen verschließt.

Im Zusammenhang mit Fig. 8 wird daher im Folgenden eine spezielle Möglichkeit erläutert, um einen Einlassschalldämpfer 21 für einen erfindungsgemäß ausgeführten Verdichter zu realisieren. Die zuvor beschriebene Funktionalität wird hierbei zumindest näherungsweise dadurch erreicht, dass ein Einlassschalldämpfer 21 wenigstens ein selbsttätig schließendes Ventilelement 23 aufweist, das in Abhängigkeit der am Ventil anliegenden Druckdifferenzen öffnet und schließt. Die Betätigung des Ventilelements 23 erfolgt hierbei bevorzugt aufgrund der am Ventilteller 24 anliegenden Druckdifferenz. Das Ventilelement 23 ist daher auf vorteilhafte Weise in Form eines Rückschlagventils ausgeführt, das ein Rückströmen von Luft- und/oder Gas aus dem Strömungskanal des Verdichterrades 6 zuverlässig verhindert. Das in Fig. 8 gezeigte Ventilelement verfügt über einen Ventilteller 24, der eine Ventilmembran 28 aufweist.

Die erforderliche Öffnungscharakteristik wird dadurch eingestellt, dass die Steifigkeit der verwendeten Ventilmembran 28, ihre Form, ihre Anordnung im Strömungskanal und/oder ihre Befestigung einer Ventilmembran bedarfsgerecht gewählt wird.

Das gezeigte Ventilelement 24 eines Einlassschalldämpfers 21 ist derart ausgeführt, dass es eine maximale Ventilöffnung 26 für einen maximalen Strömungsquerschnitt zwischen dem Ventilteller 24 und dem Ventilsitz 25 bei maximalem Volumenstrom, also bei maximaler pneumatischen Leistung des Verdichters, und eine minimale Ventilöffnung 26 für einen minimalen Strömungsquerschnitt bei maximal gedrosseltem Betrieb des Verdichters freigibt. Hierdurch wird während des Betriebs eine maximal mögliche Minimierung des durch den Einlass emittierten Schalls und damit eine maximale Schalldämmung erreicht.

In diesem Zusammenhang zeigt Fig. 8 a) einen Betriebszustand, in dem das Ventilelement 23 geöffnet und der Ventilteller 24 vom Ventilsitz 25 abgehoben ist, sodass die Ventilöffnung 26 wenigstens teilweise freigegeben ist. Demgegenüber zeigt Fig. 8 b) einen Betriebszustand, in dem das Ventilelement 24 verschlossen ist und der Ventilteller 25 entlang seines äußeren Umfangs am Ventilsitz 26 anliegt.

Grundsätzlich ist es denkbar, in einem Einlassschalldämpfer 21 ein Ventilelement 24, wie es zuvor beschrieben wurde, vorzusehen oder wenigstens zwei derartige Ventilelemente zu verwenden. Werden eine Mehrzahl derartiger Ventilelemente 24 mit unterschiedlichen Öffnungscharakteristika in Reihe geschaltet, kann die Kennlinie des Verdichters bedarfsgerecht optimiert werden, sodass beispielsweise eine konstante Verdichtung über einen vorgegebenen Volumenstrombereich realisiert werden kann.

Ebenso kann wenigstens eines der zuvor beschriebenen Ventilelemente 24 allein oder gemeinsam mit einem Spiralschalldämpfer, wie er in den Figuren 6 und 7 gezeigt ist, zur Minimierung des über den Einlass 7 austretenden Schalls eingesetzt werden, insbesondere um Schallemissionen, die vom Verdichterrad 6 erzeugt und entgegen der Strömungsrichtung des geförderten Luft- und/oder Gasstroms über den Einlass 8 austreten würden, zu verhindern oder zumindest zu verringern. Diese Schallemissionen über den Einlass 8 werden üblicherweise stärker, sofern das Verdichterrad 6 auf einem gleichbleibenden Drehzahlniveau gedrosselt wird. Hieraus folgt, dass in einem Betriebspunkt, in dem der Verdichter nicht durchströmt wird, die Schallemission einen maximalen Wert annimmt.

### Bezugszeichenliste

- 1: Verdichter
- 2: Sammelgehäuse
- 3: Gehäuseinnenraum
- 4: Antriebswelle
- 5: Elektromotor
- 6: Verdichterrad
- 7: Saugseite
- 8: Einlass
- 9: Druckseite
- 10: Auslass
- 11: Strömungskanal
- 12: Deckelelement
- 13: Spalt
- 14: Funktionsbauteil
- 15: Entkopplungselement
- 16: Umgebung
- 17: Strömungsführungseinheit
- 18: Leitschaufel
- 19: Hauptschaufel
- 20: Zwischenschaufel
20a längere Zwischenschaufel
20b kürzere Zwischenschaufel
- 21: Einlassschalldämpfer
- 22: Spiralelement
- 23: Ventilelement
- 24: Ventilteller
- 25: Ventilsitz
- 26: Ventilöffnung
- 27: Sekundärgehäuse
- 28: Ventilmembran
- 29: Schraube

## Patentansprüche

1. Verdichter (1) zur Förderung eines Luft- und/oder Gasstroms für ein Beatmungs-oder Anästhesiegerät, mit einem Gehäuse (2), in dessen Gehäuseinnenraum (3) ein drehbar gelagertes und über eine Antriebswelle (4) mit einem Elektromotor (5) verbundenes Verdichterrad (6) angeordnet ist, bei dessen Drehung der Luft- und/oder Gasstrom von einem stromaufwärts auf einer Saugseite (7) des Verdichterrades (6) angeordneten Einlass (8) durch einen Strömungskanal (11) zu einem stromabwärts auf einer Druckseite (9) des Verdichterrades (6) angeordneten Auslass (10) gefördert wird, wobei das im Gehäuseinnenraum (3) angeordnete Verdichterrad (6) zumindest bereichsweise von dem Gehäuse, das als Sammelgehäuse (2) für den aus dem Verdichterrad (6) austretenden Luft- und/oder Gasstrom ausgeführt und zumindest teilweise auf der Druckseite (9) des Verdichterrades (6) angeordnet ist, und von einem Deckelelement (12), das zumindest teilweise auf der Saugseite (7) des Verdichterrades (6) angeordnet und wenigstens abschnittsweise durch einen Spalt (13) vom Verdichterrad (6) getrennt ist, umgeben ist, wobei zwischen dem Deckelelement (12) und dem Sammelgehäuse (2) und zwischen einem mit dem Verdichterrad (6) und/oder dem Elektromotor (5) zumindest mittelbar verbundenen Funktionsbauteil (14) und dem Sammelgehäuse (2) jeweils wenigstens ein Entkopplungselement (15) zur Schwingungsdämpfung und wenigstens teilweisen Abdichtung des Gehäuseinnenraums (3) gegenüber einer Umgebung (16) angeordnet sind, **dadurch gekennzeichnet, dass** das Sammelgehäuse (2) spiralförmig ausgeführt ist.

2. Verdichter nach Anspruch 1,
**dadurch gekennzeichnet, dass** das Funktionsbauteil (14) Teil eines Gehäuses und/oder einer Kapselung des Elektromotors (5) ist und/oder mittelbar oder unmittelbar mit dem Elektromotor (5) verbunden ist.

3. Verdichter nach Anspruch 1 oder 2,
**dadurch gekennzeichnet, dass** das Deckelelement (12) in Form einer Scheibe ausgeführt ist, die im Scheibeninneren einen Durchlass für die Luft- und/oder Gasströmung aufweist.

4. Verdichter nach wenigstens einem der vorangehenden Ansprüche,
**dadurch gekennzeichnet, dass** das Deckelelement (12) wenigstens bereichsweise mittelbar oder unmittelbar mit einer Strömungsführungseinheit (17) verbunden ist, die über wenigstens eine Leitschaufel (18) zur Führung der auf der Druckseite (9) des Verdichterrades (6) austretenden Luft- und/oder Gasströmung verfügt.

5. Verdichter nach Anspruch 4,
**dadurch gekennzeichnet, dass** die Strömungsführungseinheit (17) ringförmig ausgebildet ist, über eine Mehrzahl auf einer Ringfläche angeordnete Leitschaufeln (18) verfügt und dass das Verdichterrad (6) derart im Inneren der Strömungsführungseinheit (17) angeordnet ist, dass die aus dem Verdichterrad (6) auf der Druckseite (9) austretende Luft- und/oder Gasstrom wenigstens teilweise auf die Mehrzahl von Leitschaufeln (18) auftrifft.

6. Verdichter nach wenigstens einem der vorangehenden Ansprüche,
**dadurch gekennzeichnet, dass** das Verdichterrad (6) über sich in radialer Richtung vom Außenumfang des Verdichterrades (6) quer über zumindest weitgehend einen gesamten Querschnitt des Strömungskanals (11) erstreckende Hauptschaufeln (19) sowie jeweils wenigstens zwei zwischen den Hauptschaufeln (19) angeordnete und kürzer als die Hauptschaufeln ausgeführte Zwischenschaufeln (20) verfügt.

7. Verdichter nach Anspruch 6,
**dadurch gekennzeichnet, dass** die zwischen zwei Hauptschaufeln (19) angeordneten Zwischenschaufeln (20) in radialer Richtung unterschiedlich lang sind, über ein ungleiches Profil verfügen und/oder verschieden große Schaufelflächen aufweisen.

8. Verdichter nach wenigstens einem der vorangehenden Ansprüche,
**dadurch gekennzeichnet, dass** stromaufwärts des Verdichterrades (6) wenigstens ein Einlassschalldämpfer (21) angeordnet ist.

9. Verdichter nach Anspruch 8,
**dadurch gekennzeichnet, dass** der Einlassschalldämpfer (21) als Spiralschalldämpfer ausgeführt ist.

10. Verdichter nach Anspruch 8 oder 9,
**dadurch gekennzeichnet, dass** der Einlassschalldämpfer (21) als Spiralschalldämpfer mit wenigstens zwei zumindest teilweise ineinander angeordneten separaten Spiralelementen (22) ausgeführt ist.

11. Verdichter nach wenigstens einem der vorangehenden Ansprüche,
**dadurch gekennzeichnet, dass** stromaufwärts des Verdichterrades (6) wenigstens ein sich aufgrund einer durch Drehung des Verdichterrades (6) verursachten Druckdifferenz in Strömungsrichtung des Luft- und/oder Gasstroms öffnendes Ventilelement (23) angeordnet ist.

12. Verdichter nach Anspruch 11,
**dadurch gekennzeichnet, dass** das Ventilelement (23) als Ventilteller (24) eine bewegbar gelagerte Ventilmembran aufweist.

13. Beatmungs- oder Anästhesiegerät mit einem Verdichter (1) nach wenigstens einem der vorangehenden Ansprüche.

## Claims

1. Compressor (1) for conveying an air and/or gas stream for a ventilator or anaesthesia machine, having a housing (2), in the housing interior space (3) of which there is arranged a rotatably mounted compressor wheel (6) which is connected to an electric motor (5) via a drive shaft (4), during the rotation of which compressor wheel the air and/or gas stream is conveyed from an inlet (8) arranged upstream on a suction side (7) of the compressor wheel (6) to an outlet (10) arranged downstream on a pressure side (9) of the compressor wheel (6) through a flow channel (11), wherein the compressor wheel (6) arranged in the housing interior space (3) is at least regionally surrounded by the housing, which is in the form of a collecting housing (2) for the air and/or gas stream exiting the compressor wheel (6) and is at least partially arranged on the pressure side (9) of the compressor wheel (6), and by a cover element (12), which is at least partially arranged on the suction side (7) of the compressor wheel (6) and is at least sectionally separated from the compressor wheel (6) by a gap (13), wherein, between the cover element (12) and the collecting housing (2) and between a functional component (14), which is at least indirectly connected to the compressor wheel (6) and/or the electric motor (5), and the collecting housing (2), there is arranged in each case at least one decoupling element (15) for vibration damping and at least partial sealing of the housing interior space (3) with respect to surroundings (16), **characterized in that** the collecting housing (2) is of spiral-shaped design.

2. Compressor according to Claim 1,
**characterized in that** the functional component (14) is part of a housing and/or of an encapsulation of the electric motor (5) and/or is indirectly or directly connected to the electric motor (5).

3. Compressor according to Claim 1 or 2,
**characterized in that** the cover element (12) is in the form of a disc which, in the disc interior, has a passage for the air and/or gas flow.

4. Compressor according to at least one of the preceding claims,
**characterized in that** the cover element (12) is at least regionally indirectly or directly connected to a flow-guiding unit (17) which has at least one guide blade (18) for guiding the air and/or gas flow exiting on the pressure side (9) of the compressor wheel (6).

5. Compressor according to Claim 4,
**characterized in that** the flow-guiding unit (17) is of annular form and has a plurality of guide blades (18) which are arranged on an annular surface, and **in that** the compressor wheel (6) is arranged in the interior of the flow-guiding unit (17) in such a way that the air and/or gas stream exiting the compressor wheel (6) on the pressure side (9) at least partially impinges on the plurality of guide blades (18).

6. Compressor according to one of the preceding claims, **characterized in that** the compressor wheel (6) has main blades (19) extending in a radial direction from the outer circumference of the compressor wheel (6) transversely over at least largely an entire cross section of the flow channel (11), and has at least two intermediate blades (20) arranged between the main blades (19) in each case, which are designed to be shorter than the main blades.

7. Compressor according to Claim 6,
**characterized in that** the intermediate blades (20) arranged between two main blades (19) are radially of different length, have an unlike profile, and/or have blade surfaces of different sizes.

8. Compressor according to one of the preceding claims, **characterized in that** at least one inlet sound damper (21) is arranged upstream of the compressor wheel (6).

9. Compressor according to Claim 8,
**characterized in that** the inlet sound damper (21) is in the form of a spiral sound damper.

10. Compressor according to Claim 8 or 9,
**characterized in that** the inlet sound damper (21) is in the form of a spiral sound damper with at least two separate spiral elements (22) which are at least partially arranged one in the other.

11. Compressor according to at least one of the preceding claims,
**characterized in that**, upstream of the compressor wheel (6), there is arranged at least one valve element (23) which opens owing to a pressure difference, caused by rotation of the compressor wheel (6), in the flow direction of the air and/or gas stream.

12. Compressor according to Claim 11,
**characterized in that** the valve element (23) has a movably mounted valve diaphragm as a valve plate (24).

13. Ventilator or anaesthesia machine having a compressor (1) according to at least one of the preceding claims.

## Revendications

1. Compresseur (1) pour transporter un écoulement d'air et/ou de gaz pour un appareil de respiration ou d'anesthésie, comprenant un boîtier (2) dans l'espace intérieur de boîtier (3) duquel est agencée une roue de compresseur (6) montée de manière rotative et reliée à un moteur électrique (5) par l'intermédiaire d'un arbre d'entraînement (4), par la rotation de laquelle l'écoulement d'air et/ou l'écoulement de gaz est acheminé à partir d'une entrée (8) agencée en amont sur un côté d'aspiration (7) de la roue de compresseur (6) à travers un canal d'écoulement (11) jusqu'à une sortie (10) agencée en aval sur un côté de pression (8) de la roue de compresseur (6), dans lequel la roue de compresseur (6) agencée dans l'espace intérieur de boîtier (3) est entourée, au moins partiellement par le boîtier, qui se présente sous forme d'un boîtier de collecte (2) pour l'écoulement d'air et/ou de gaz sortant de la roue de compresseur (6) et qui est agencé, au moins partiellement, sur le côté de pression (9) de la roue de compresseur (6), et qui est entouré par un élément de capot (12) qui est agencé, au moins partiellement, sur le côté d'aspiration (7) de la roue de compresseur (6) et qui est séparé, au moins partiellement, de la roue de compresseur (6) par un espace (13), dans lequel respectivement entre l'élément de capot (12) et le boîtier de collecte (2) et entre un composant fonctionnel (14), relié au moins indirectement à la roue de compresseur (6) et/ou au moteur électrique (5), et le boîtier de collecte (2) sont agencés au moins un élément de découplage (15) servant à amortir les vibrations et à rendre étanche au moins par endroits l'espace intérieur de boîtier (3) vis-à-vis d'un environnement (16), **caractérisé en ce que** le boîtier de collecte (2) est conçu en forme de spirale.

2. Compresseur selon la revendication 1, **caractérisé en ce que** le composant fonctionnel (14) fait partie d'un boîtier et/ou d'un encapsulage du moteur électrique (5) et/ou est relié directement ou indirectement au moteur électrique (5).

3. Compresseur selon la revendication 1 ou 2, **caractérisé en ce que** l'élément de capot (12) est réalisé sous la forme d'un disque qui présente à l'intérieur du disque un passage pour l'écoulement d'air et/ou de gaz.

4. Compresseur selon au moins l'une quelconque des revendications précédentes, **caractérisé en ce que** l'élément de capot (12) est relié au moins par endroits directement ou indirectement à une unité de guidage d'écoulement (17) qui dispose d'au moins une aube directrice (18) pour guider l'écoulement d'air et/ou de gaz sortant sur le côté de pression (9) de la roue de compresseur (6).

5. Compresseur selon la revendication 4, **caractérisé en ce que** l'unité de guidage d'écoulement (17) est réalisée avec une forme annulaire, dispose d'une pluralité d'aubes directrices (18) agencées sur une surface annulaire, et **en ce que** la roue de compresseur (6) est agencée à l'intérieur de l'unité de guidage d'écoulement (17) de telle sorte que l'écoulement d'air et/ou de gaz sortant de la roue de compresseur (6) sur le côté de pression (9) heurte au moins partiellement la pluralité d'aubes directrices (18).

6. Compresseur selon au moins l'une des revendications précédentes, **caractérisé en ce que** la roue de compresseur (6) dispose d'aubes principales (19) s'étendant dans la direction radiale à partir de la circonférence extérieure de la roue de compresseur (6), transversalement au moins dans une large mesure à toute une section transversale du canal d'écoulement (11), ainsi que d'au moins deux aubes intermédiaires (20) agencées entre les aubes principales (19) et plus courtes que les aubes principales.

7. Compresseur selon la revendication 6, **caractérisé en ce que** les aubes intermédiaires (20) agencées entre deux pales principales (19) sont de longueur différente dans la direction radiale, présentent un profil inégal et/ou présentent des surfaces d'aube de tailles différentes.

8. Compresseur selon au moins l'une quelconque des revendications précédentes, **caractérisé en ce qu'**au moins un silencieux d'admission (21) est agencé en amont de la roue de compresseur (6).

9. Compresseur selon la revendication 8, **caractérisé en ce que** le silencieux d'admission (21) est réalisé sous la forme d'un silencieux en spirale.

10. Compresseur selon la revendication 8 ou 9, **caractérisé en ce que** le silencieux d'admission (21) est réalisé sous la forme d'un silencieux en spirale avec au moins deux éléments en spirale (22) séparés, agencés au moins en partie l'un dans l'autre.

11. Compresseur selon au moins l'une quelconque des revendications précédentes, **caractérisé en ce qu'**en amont de la roue de compresseur (6) est agencé au moins un élément de soupape (23) s'ouvrant dans la direction d'écoulement de l'écoulement d'air et/ou de gaz en raison d'une différence de pression provoquée par la rotation de la roue de compresseur (6).

12. Compresseur selon la revendication 11, **caractérisé en ce que** l'élément de soupape (23) présente, en tant que disque de soupape (24), une membrane de soupape montée de manière mobile.

13. Ventilateur ou machine d'anesthésie avec un compresseur (1) selon au moins l'une quelconque des revendications précédentes.
